(19) Europäisches Patentamt
European Patent Office
Office européen des brevets

(11) Veröffentlichungsnummer: **0 039 795**
**B1**

(12) # EUROPÄISCHE PATENTSCHRIFT

(45) Veröffentlichungstag der Patentschrift: **12.10.83**

(51) Int. Cl.³: **C 07 D 275/04**

(21) Anmeldenummer: **81102875.2**

(22) Anmeldetag: **15.04.81**

(54) **Verfahren zur Herstellung von 1,2-Benzisothiazolen.**

(30) Priorität: **12.05.80 DE 3018108**

(43) Veröffentlichungstag der Anmeldung: **18.11.81 Patentblatt 81/46**

(45) Bekanntmachung des Hinweises auf die Patenterteilung: **12.10.83 Patentblatt 83/41**

(84) Benannte Vertragsstaaten: **BE CH DE FR GB LI**

(56) Entgegenhaltungen:
**CHEMICAL ABSTRACTS, vol 58, no. 11, May 27, 1963 A. RICCI «A new synthesis of benzisothiazoles» abstract no. 11340c**

(73) Patentinhaber: **BASF Aktiengesellschaft, Carl-Bosch-Strasse 38, D-6700 Ludwigshafen (DE)**

(72) Erfinder: **Hagen, Helmut, Dr., Max-Slevogt-Strasse 17E, D-6710 Frankenthal (DE)**
Erfinder: **Markert, Juergen, Dr., Am Speyerweg 26, D-6704 Mutterstadt (DE)**
Erfinder: **Ziegler, Hans, Dr., Am Speyerweg 48, D-6704 Mutterstadt (DE)**

**EP 0 039 795 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

# Verfahren zur Herstellung von 1,2-Benzisothiazolen

Die Erfindung betrifft ein Verfahren zur Herstellung von 1,2-Benzisothiazolen durch Umsetzung von o-Halogenarylmethylverbindungen mit 18 bis 31-gewichtsprozentigen, wässrigen Ammoniaklösungen und elementarem Schwefel bei Drücken von 0,8 bis 1,2 bar und Temperaturen von 65 bis 110 °C in Gegenwart von disubstituierten Sulfoxiden, Sulfonen und/oder Formamiden als Lösungsmittel.

Es ist aus der Angewandten Chemie, Band 36, Seite 159 (1923) und aus den Berichten der deutschen Chemischen Gesellschaft, Band 58, Seite 2095 (1925) bekannt, Thionaphthen-2,3-dion mit Ammoniak und Wasserstoffperoxid zu 3-Carbamyl-1,2-benzisothiazol umzusetzen und daraus durch Hydrolyse und Decarboxylierung 1,2-Benzisothiazol zu gewinnen. Die vorgenannten Berichte, Band 56, Seite 1630 (1923) und Liebigs Annalen der Chemie, Band 454, Seite 264 (1927) beschreiben die Umsetzung von 2-Formyl-4-nitrophenylsulfenylbromid mit Ammoniak zu 5-Nitro-1,2-benzisothiazol. Ebenfalls kann man Benzisothiazole durch Cyclisierung von o-Mercapto-phenylcarbonyl-verbindungen in Gegenwart von Polyphosphorsäure synthetisieren (Annali die Chimica, Band 53, Nummer 5, Seiten 577 bis 587 (1963)).

Es ist aus der deutschen Offenlegungsschrift 1 670 196 bekannt, dass man Dihalogenmethylarylverbindungen mit Ammoniak und Schwefel zu Benzisothiazolen umsetzt. Ammoniak wird als Gas und vorzugsweise im Überschuss angewendet. Wie die Beispiele zeigen, setzt man unter Druck während 5 bis 10 Stunden bei 100 bis 150 °C um. Als Lösungsmittel werden Benzol und Methanol verwendet.

Es ist aus der deutschen Offenlegungsschrift 25 03 699 ein Verfahren durch Umsetzung von o-Halogenarylaldehyden mit Ammoniak und elementarem Schwefel bekannt. Auch hier wird lediglich mit Ammoniakgas und in der Regel unter Druck umgesetzt. Im allgemeinen verwendet man Glykolmonoäther, insbesondere Glykolmonomethyläther, und Alkanole, insbesondere Methanol, als Lösungsmittel. In der Beschreibung werden neben Alkanolen und Äthern nur noch aromatische Kohlenwasserstoffe als Lösungsmittel aufgezählt. Lediglich Beispiel 2 zeigt eine drucklose Umsetzung bei 20 bis 25 °C mit einer Ausbeute von 84% der Theorie, wofür aber eine Reaktionszeit von 25 Stunden benötigt wird.

In der deutschen Offenlegungsschrift 27 34 866 wird die Umsetzung von o-Halogenarylketonen mit Ammoniak und elementarem Schwefel beschrieben. Auch diese Veröffentlichung lehrt eine Reaktion mit Ammoniakgas unter Druck in Gegenwart vorgenannter organischer Lösungsmittel. Bevorzugt werden, wie alle Beispiele zeigen, Methanol, Glykolmonomethyläther und der entsprechende Monoäthyläther verwendet.

Alle diese Verfahren sind mit Bezug auf leicht zugängliche Ausgangsstoffe, Wirtschaftlichkeit, Einfachheit des Betriebs bei gleichzeitig guter Ausbeute an Endstoff unbefriedigend.

Es wurde nun gefunden, dass man 1,2-Benzisothiazole der Formel

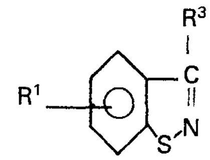

I,

worin $R^1$ Wasserstoff, einen aliphatischen, cycloaliphatischen oder aromatischen Rest, einen anellierten Phenylenrest, Halogen, eine Alkoxygruppe ,Nitrogruppe, oder den Rest $-N\begin{smallmatrix}R^2\\R^2,\end{smallmatrix}$ worin die einzelnen Reste $R^2$ gleich oder verschieden sein können und jeweils Wasserstoff, einen aliphatischen, cycloaliphatischen, araliphatischen oder aromatischen Rest bedeuten, bezeichnet, und $R^3$ für Wasserstoff, einen aromatischen oder heterocyclischen Rest steht, durch Umsetzung von o-Halogenarylmethylverbindungen der Formel

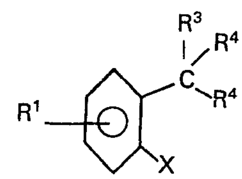

II,

in der X für Halogen steht, $R^1$ und $R^3$ die vorgenannte Bedeutung haben, die beiden Reste $R^4$ gleich oder verschieden sein können und jeweils ein Halogenatom bezeichnen oder zusammen für eine Oxogruppe stehen, mit Ammoniak und elementarem Schwefel in Gegenwart eines organischen Lösungsmittels bei erhöhter Temperatur vorteilhaft erhält, wenn die Umsetzung mit 18- bis 31-gewichtsprozentigen, wässrigen Ammoniaklösungen bei Drücken von 0,8 bis 1,2 bar und Temperaturen von 65 bis 110 °C in Gegenwart von disubstituierten Sulfoxiden, Sulfonen und/oder Formamiden als Lösungsmittel durchgeführt wird.

Die Reaktion lässt sich am Beispiel der Umsetzung von 2-Chlor-5-nitro-benzaldehyd mit wässrigem Ammoniak und Schwefel formelmässig wie folgt wiedergeben:

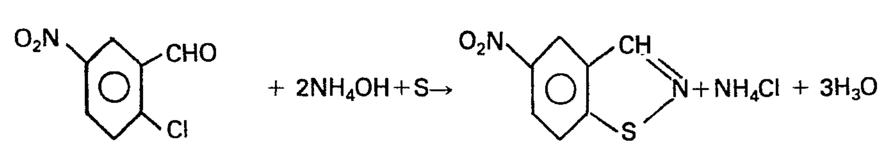

Am Beispiel von 2,6-Dichlorbenzalchlorid lässt sich die Umsetzung formelmässig wie folgt wiedergeben:

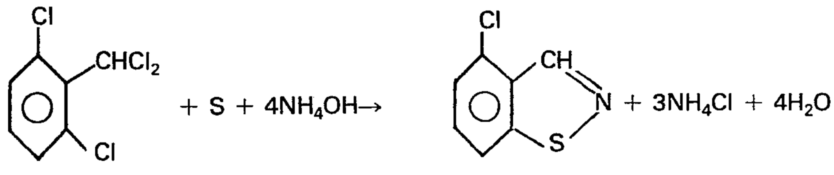

Da beide in o-Stellung zur Dihalogenmethylgruppe befindlichen Chloratome reaktiv sind, kann sich der Isothiazolring auch zu der entgegengesetzten o-Stellung hin schliessen; bei entsprechend substituierten Stoffen II kann man gegebenenfalls so Gemische der 2 entsprechenden Benzisothiazole erhalten.

Im Vergleich zu den erstgenannten, bekannten Verfahren geht das Verfahren nach der Erfindung von leichter zugänglichen Ausgangsstoffen aus und liefert überraschend auf einfache und wirtschaftliche Weise 1,2-Benzisothiazole in besserer Ausbeute und Reinheit. Im Hinblick auf die deutschen Offenlegungsschriften 16 70 196, 25 03 699 und 27 34 866 können o-Halogenarylmethylverbindungen drucklos bzw. mit leichtem Unterdruck oder geringerem Überdruck umgesetzt werden. Druckapparaturen und entsprechende Schwierigkeiten des Betriebs mit Ammoniak unter Druck werden vermieden, Abgas- und Abwasserprobleme wesentlich verringert. Vorteilhaft können auch feuchte oder wasserhaltige Reaktionskomponenten, z.B. feuchte Ausgangsstoffe II, verwendet werden. Gegenüber der Verfahrensweise, wie es Beispiel 2 der deutschen Offenlegungsschrift 25 03 699 zeigt, besitzt das erfindungsgemässe Verfahren, wie z.B. das erfindungsgemässe Beispiel 1 zeigt, eine höhere Reaktionsgeschwindigkeit, wesentlich verringerte Reaktionszeit und somit bei gleichzeitig höherer Ausbeute eine wesentlich bessere Raum-Zeit-Ausbeute. Im Vergleich zu der Arbeitsweise der 3 letztgenannten deutschen Offenlegungsschriften unter Druck ist das erfindungsgemässe Verfahren rascher durchführbar, besitzt eine bessere Raum-Zeit-Ausbeute und häufig eine bessere Gesamtausbeute und Reinheit des Endstoffs. Die bessere Raum-Zeit-Ausbeute wird in einigen Fällen bei dem erfindungsgemässen Verfahren auch dadurch erzielt, dass drucklos und bei tieferer Temperatur umgesetzt wird und dementsprechende Operationszeit zur Einstellung höherer Drücke und Temperaturen bzw. zum Abkühlen und für die Entnahme des Reaktionsgemischs eingespart wird. Alle diese vorteilhaften Ergebnisse des erfindungsgemässen Verfahrens sind im Hinblick auf den Stand der Technik überraschend.

Bevorzugte Ausgangsstoffe II und dementsprechend bevorzugte 1,2-Benzisothiazole I sind solche, in deren Formeln $R^1$ einen gegebenenfalls durch eine Carbonamidogruppe substituierten Alkylrest mit 1 bis 6 Kohlenstoffatomen, einen Cycloalkylrest mit 5 bis 8 Kohlenstoffatomen, einen Phenylrest oder Naphthylrest, einen anellierten Phenylenrest, Wasserstoff, Brom oder insbesondere Chlor, eine Alkoxygruppe mit 1 bis 4 Kohlenstoffatomen, eine Nitrogruppe oder den Rest

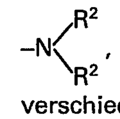

, worin die einzelnen Reste $R^2$ gleich oder verschieden sein können und jeweils Wasserstoff, einen Alkylrest mit 1 bis 6 Kohlenstoffatomen, einen Alkylenrest mit 3 bis 6 Kohlenstoffatomen, einen Cycloalkylrest mit 5 bis 8 Kohlenstoffatomen, einen Aralkylrest oder Alkylarylrest mit jeweils 7 bis 12 Kohlenstoffatomen, einen Phenylrest bedeuten, bezeichnet und $R^3$ für Wasserstoff, einen unsubstituierten oder einen durch ein oder mehrere, vorzugsweise ein oder zwei Alkylreste, vorzugsweise mit 1 bis 4 Kohlenstoffatomen, Alkoxygruppen, vorzugsweise mit 1 bis 4 Kohlenstoffatomen, Chloratome, Bromatome, Dialkylaminogruppen, vorzugsweise mit 1 bis 4 Kohlenstoffatomen je Alkylgruppe, substituierten Phenylrest oder Naphthylrest oder einen 5- oder 6-gliedrigen, heterocyclischen Ring, der zwei Stickstoffatome oder ein Stickstoffatom, ein Sauerstoffatom und/oder ein Schwefelatom enthalten kann, steht, die beiden Reste $R^4$ gleich oder verschieden sein können und jeweils ein Bromatom oder zweckmässig ein Chloratom bezeichnen oder zusammen für eine Oxogruppe stehen, X Brom oder insbesondere Chlor bedeutet. Die vorgenannten Reste können noch durch unter den Reaktionsbedingungen inerte Gruppen, z.B. Alkylgruppen oder Alkoxygruppen mit jeweils 1 bis 4 Kohlenstoffatomen, Nitrogruppen, Carbonamidogruppen, substituiert sein. Statt der Ausgangsstoffe II können auch Stoffe, die die Ausgangsstoffe II unter den Reaktionsbedingungen bilden, z.B. die entsprechenden Aldehydacetale oder Ketonacetale wie o-Chlorbenzophenon-dimethylacetal, verwendet werden. Ausgangsstoffe II, Ammoniak und elementarer Schwefel können in etwa stöchiometrischen Mengen verwendet werden, jedoch verwendet man vorzugsweise ein Verhältnis von 5 bis 40, insbesondere 8 bis 15 Mol Ammoniak und/oder von 0,8 bis 1,5, insbesondere 0,9 bis 1,1 Grammatom Schwefel je Mol Ausgangsstoff II.

Als Ausgangsstoffe II kommen beispielsweise in Betracht: 5-Nitro-, 4-Dimethylamino-, 4-Diäthylamino-, 4-Diallylamino-, 4-Di-(2'-methylallyl)-amino-, 4-(N-Methyl-N-2-carbonamidoäthylamino)-, 6-Methyl-, 3-Äthyl-, 5-Hexyl-, 6-Isobutyl-, 5-Propyl-, 4-tert.-Butyl-, 4-Cyclohexyl-, 4-Cyclopentyl-, 5-Phenyl-, 4-Phenyl-, 4-Nitrophenyl-, 4-o-Toluyl-, 4-p-Äthoxyphenyl-, 3-Di-(2'-äthoxyäthyl)-amino-, 4-Naphthyl-, 4-Brom-, 6-Methoxy-, 6-Dicyclohexylamino-, 4-Dibenzylamino-, 4-Diphenylamino-, 4-p-Xylyl-2-chlorbenzophenon, 2-Chlorbenzophenon, 2-Brombenzophenon; 1-Chlor-2-benzoyl-, 2-Chlor-3-benzoyl-, 2-Chlor-1-benzoylnaphthalin; entsprechend substituierte Bromarylketone; analog mit vorgenannten Substituenten an beiden Phenylkernen substituierte Benzophenone; analoge Aroylbenzole, deren Aroylrest mit Chlor oder Brom substituiert ist und die am Benzolkern in o-, m- und/oder p-Stellung ein oder zwei Methyl-, Äthyl-, Propyl-, Isopropyl-, Butyl-, Isobutyl-, sek.-Butyl-, tert.-Butyl-, Chlor-, Brom-, Äthoxy-, Methoxy-, Propoxy-, Isopropoxy-, Butoxy-, Dimethylamino-, Diäthylamino-, Dipropylamino-, Diisopropylamino-gruppen tragen; mit vorgenannten Substituenten am Aroylkern substituierte Aroylnaphthaline, Aroylthiophene, Aroyltetrahydrofurane, Aroylpyrane und entsprechende Aroylverbindungen von Imidazol, 1-Methylimidazol, 1-Propylimidazol, 2-Methylpyridin, 3-Methylpyridin, 4-Methylpyridin, 2,4-Dimethylpyridin, 2,6-Dimethylpyridin, 2,4,6-Trimethylpyridin, Pyridin, Pyrrolidin, Pyrrol, Imidazolidin, Piperidin, Morpholin, Pyridazin, Pyrimidin, Pyrazin, Piperazin; 5-Nitro-, 4-Dimethylamino-, 4-Diäthylamino-, 4-Diallylamino-, 4-Di-(2'-methylallyl)-amino-, 4-(N-Methyl-N-2-carbonamidoäthylamino)-, 6-Methyl-, 3-Äthyl-, 5-Hexyl-, 6-Isobutyl-, 5-Propyl-, 4-tert.-Butyl-, 4-Cyclohexyl-, 4-Cyclopentyl-, 5-Phenyl, 4-Phenyl-, 4-Nitrophenyl-, 4-p-Toluyl-, 4-p-Äthoxyphenyl-, 3-Di-(2'-äthoxyäthyl)-amino-, 4-Naphthyl-, 4-Brom-, 6-Methoxy-, 6-Dicyclohexylamino-, 4-Dibenzylamino-, 4-Diphenylamino-, 4-p-Xylyl-2-chlorbenzaldehyd; 2-Chlorbenzaldehyd, 2-Brombenzaldehyd; 1-Chlor-2-formyl-, 2-Chlor-3-formyl-, 2-Chlor-1-formyl-naphthalin; entsprechend substituierte Bromaryladehyde; entsprechende unsubstituierte oder substituierte o-Halogenarylmethyldibromide, -chloride.

Die Umsetzung wird bei einer Temperatur von 65 bis 110 °C, vorteilhaft von 70 bis 105 °C, insbesondere 80 bis 105 °C, drucklos oder unter geringem Überdruck oder Unterdruck, bei 0,8 bis 1,2, vorzugsweise 0,9 bis 1,1 bar, kontinuierlich oder diskontinuierlich durchgeführt. Die Umsetzung wird mit 18- bis 31-gewichtsprozentiger, vorzugsweise 20- bis 26-gewichtsprozentiger, wässriger Ammoniaklösung durchgeführt. Anstelle der Ammoniaklösung können auch getrennt voneinander eine konzentriertere Ammoniaklösung und Wasser, z.B. in Gestalt des wasserhaltigen Ausgangsstoffes II, verwendet werden. Man kann weniger zweckmässig die Ammoniaklösung auch in situ zubereiten, z.B. im Ausgangsgemisch Wasser vorlegen und entsprechend Ammoniakgas bis zu der erfindungsgemässen Konzentration einleiten. Vorteilhaft ist ein Verhältnis von 20 bis 80, vorzugsweise 40 bis 60 Gewichtsprozent Wasser, bezogen auf Sulfon, Sulfoxid und/oder Formamid.

Als Lösungsmittel werden vorteilhaft solche der Formel

$$R^5\text{–}Y\text{–}R^6 \qquad \text{III}$$

gewählt, worin $R^5$ und $R^6$ gleich oder verschieden sind und jeweils einen aliphatischen Rest, vorzugsweise einen Alkylrest mit 1 bis 8, insbesondere 1 bis 4 Kohlenstoffatomen oder $R^5$ und $R^6$ zusammen einen aliphatischen Rest, vorzugsweise einen Alkylenrest oder Alkenylenrest mit 4 bis 12, insbesondere 4 bis 6 Kohlenstoffatomen bedeuten und Y den Rest

```
                                    O
                                    ||
–N–, den Rest –S– oder den Rest – S – bezeichnet.
 |             ||                   ||
HCO            O                    O
```

Geeignete Lösungsmittel III sind z.B. Dimethylsulfoxid, Diäthylsulfoxid, Dipropylsulfoxid, Diisopropylsulfoxid, Di-n-butylsulfoxid, Diisobutylsulfoxid, Dipentylsulfoxid, Dihexylsulfoxid, Diheptylsulfoxid, Dioctylsulfoxid, Methyläthylsulfoxid, Tetramethylensulfoxid, Pentamethylensulfoxid, Dimethylsulfon, Diäthylsulfon, Dipropylsulfon, Diisopropylsulfon, Dibutylsulfon, Diisobutylsulfon, Dipentylsulfon, Dihexylsulfon, Diheptylsulfon, Dioctylsulfon, Methyläthylsulfon, Tetramethylensulfon, Pentamethylensulfon; entsprechende N-disubstituierte Formamide; besonders bevorzugt sind Tetramethylensulfon, -sulfoxid, Dimethylformamid, 3-Sulfolen. Auch Lösungsmittelgemische können verwendet werden. Zweckmässig verwendet man das Lösungsmittel in einer Menge von 200 bis 2000 Gewichtsprozent, vorzugsweise von 600 bis 1000 Gewichtsprozent, bezogen auf Ausgangsstoff II.

Die Reaktion kann wie folgt durchgeführt werden: Ausgangsstoff II, elementarer Schwefel, Ammoniaklösung und Lösungsmittel werden in einem Reaktor während 0,5 bis 6,5 Stunden bei den vorgenannten Temperaturen drucklos miteinander umgesetzt. Aus dem Reaktionsgemisch erhält man das 1,2-Benzisothiazol I nach den üblichen Verfahren, z.B. durch fraktionierte Destillation, Filtration und gegebenenfalls anschliessender Umkristallisation aus einem geeigneten Lösungsmittel, z.B. Ligroin. Man kann das Reaktionsgemisch auch nach Entfernung überflüssigen Ammoniaks und Lösungsmittels in Wasser giessen, das gebildete Gemisch mit einem geeigneten Lösungsmittel, z.B. Methylenchlorid, Benzol, extrahieren und den Extrakt in vorgenannter Weise aufarbeiten. Vorteilhaft gibt man während des Abkühlens des Reaktionsgemisches Wasser in einer Menge von mindestens der Hälfte der Menge an wässrigem Ammoniak zu, saugt den ausgefallenen Endstoff ab, wäscht mit Wasser neutral und trocknet.

Die nach dem Verfahren der Erfindung herstellbaren Verbindungen sind wertvolle Ausgangsstoffe für die Herstellung von Farbstoffen, Pflanzenschutzmitteln und Pharmazeutika. Bezüglich der Verwendung wird auf vorgenannte Veröffentlichungen verwiesen.

Die in den folgenden Beispielen angegebenen Teile sind Gewichtsteile.

Beispiel 1

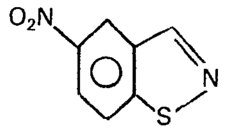

In einer Rührapparatur werden 1500 Teile 2-Chlor-5-nitrobenzaldehyd (30 Gewichtsprozent Wassergehalt) und 181 Teile Schwefel in 8000 Teilen Dimethylformamid auf 70 °C erhitzt. Bei dieser Temperatur werden innerhalb einer Stunde 4000 Teile 25-gewichtsprozentige, wässrige Ammoniaklösung langsam zugegeben. Man lässt eine Stunde bei 70 °C weiterrühren, gibt 2000 Teile Wasser hinzu, kühlt ab, saugt den ausgefallenen Feststoff ab, wäscht mit Wasser neutral und trocknet. Man erhält 983 Teile 5-Nitro-1,2-benzisothiazol mit Schmelzpunkt 151 °C. Die Ausbeute entspricht 96% der Theorie.

Beispiel 2

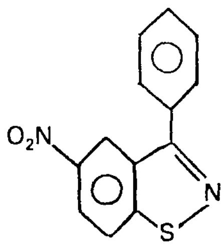

In einer Rührapparatur werden 26,2 Teile 2-Chlor-5-nitro-benzophenon und 3,2 Teile Schwefel in 160 Teilen Dimethylformamid auf 70 °C erhitzt und innerhalb von 20 Minuten 80 Teile 25-gewichtsprozentige, wässrige Ammoniaklösung langsam zugegeben. Das Gemisch wird eine Stunde bei 70 °C weitergerührt, abgekühlt und mit 80 Teilen Wasser versetzt, vom ausgefallenen Feststoff abgesaugt, der Feststoff mit Wasser neutral gewaschen und getrocknet. Man erhält 24 Teile 3-Phenyl-5-nitro-1,2-benzisothiazol mit Schmelzpunkt 135 °C. Die Ausbeute entspricht 94% der Theorie.

Beispiele 3 bis 9

Entsprechend dem Beispiel 2 werden unter denselben Bedingungen folgende Endstoffe I hergestellt.

Endstoff I

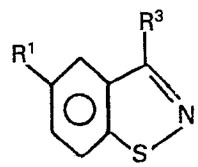

Tabelle

| | Teile | Ausgangsstoff II | $R^1$ | $R^3$ | Endstoff I Fp. in °C | Endstoff I Ausbeute (%) |
|---|---|---|---|---|---|---|
| 3 | 230.5 | 2-Chlor-5-nitro-4'-methyl-benzophenon | $NO_2$ | 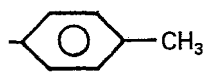 | 179 | 92 |
| 4 | 291.5 | 2-Chlor-5-nitro-4'-methoxy-benzophenon | $NO_2$ | 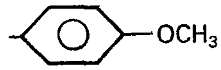 | 207 | 97,3 |
| 5 | 296 | 2-Chlor-5-nitro-4'-chlor-benzophenon | $NO_2$ | 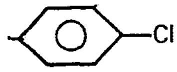 | 226 | 92 |
| 6 | 26,7 | 2-(2'-Chlor-5'-nitro-benzoyl)-thiophen | $NO_2$ | 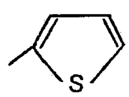 | 165 | 86 |
| 7 | 245.5 | 2-Chlor-5-nitro-2',5'-dimethyl-benzophenon | $NO_2$ | 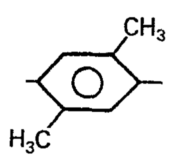 | 128 | 91 |
| 8 | 145 | 2-Chlor-5-nitro-3',4'-dimethyl-benzophenon | $NO_2$ | 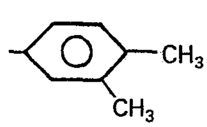 | 154 | 93 |
| 9 | 153.2 | 2-Chlor-5-nitro-3'-nitro-benzophenon | $NO_2$ | 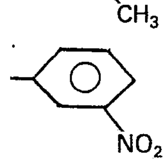 | 208 | 88 |

Beispiel 10

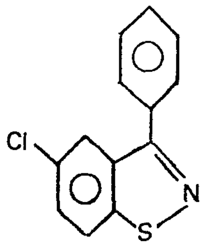

In einer Rührapparatur werden 25,1 Teile 2,5-Dichlorbenzophenon und 3,2 Teile Schwefel in 200 Teilen Dimethylformamid auf 100 °C erhitzt. Innerhalb einer Stunde werden 100 Teile 25-gewichtsprozentige, wässrige Ammoniaklösung langsam zugegeben. Das Gemisch wird anschliessend 6 Stunden bei 100 °C weitergerührt. Die Lösung wird abgekühlt, mit 100 Teilen Wasser versetzt; der ausgefallene Feststoff wird abgesaugt, mit Wasser neutral gewaschen und getrocknet. Man erhält 20 Teile 5-Chlor-3-phenyl-1,2-benzisothiazol mit Schmelzpunkt 95 °C. Die Ausbeute entspricht 81% der Theorie.

Beispiel 11

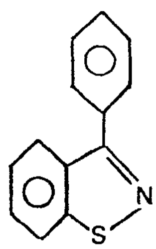

In einer Rührapparatur werden 43,3 Teile 2-Chlorbenzophenon und 6,4 Teile Schwefel in 400 Teilen Dimethylformamid auf 100 °C erhitzt und innerhalb einer Stunde 150 Teile 25-gewichtsprozentige, wässrige Ammoniaklösung langsam zugegeben. Das Gemisch wird 2 Stunden bei 100 °C weitergerührt. Die Lösung wird abgekühlt, mit 150 Teilen Wasser versetzt; der ausgefallene Feststoff wird abgesaugt, neutral gewaschen und getrocknet. Man erhält 32 Teile 3-Phenyl-1,2-benzisothiazol mit Schmelzpunkt 70 °C. Die Ausbeute beträgt 76% der Theorie.

Beispiel 12

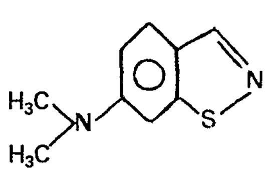

In einer Rührapparatur werden 18,3 Teile 2-Chlor-4-dimethylaminobenzaldehyd und 3,2 Teile Schwefel in 160 Teilen Dimethylformamid auf 80 °C erhitzt. 80 Teile 25-gewichtsprozentige, wässrige Ammoniaklösung werden innerhalb 30 Minuten langsam zugegeben. Das Gemisch wird 6 Stunden bei 80 °C weitergerührt, abgekühlt, mit 80 Teilen Wasser versetzt; der ausgefallene Feststoff wird abgesaugt, mit Wasser neutral gewaschen und getrocknet. Man erhält 15 Teile 6-Dimethylamino-1,2-benzisothiazol vom Schmelzpunkt 85 °C. Die Ausbeute beträgt 84% der Theorie.

Beispiel 13

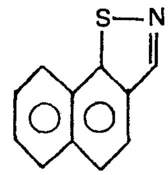

In einem Rührkessel werden 245,5 Teile 1-Chlor-2-dichlormethylnaphthalin und 32 Teile Schwefel in 1500 Teilen Dimethylformamid auf 80 °C erhitzt, 600 Teile wässrige, 25-gewichtsprozentige Ammoniaklösung werden innerhalb einer Stunde langsam zugegeben. Das Gemisch wird 2 Stunden bei 80 °C nachgerührt, abgekühlt, mit 400 Teilen Wasser versetzt; der ausgefallene Feststoff wird abgesaugt, mit Wasser neutral gewaschen und getrocknet. Man erhält 145 Teile Naphtho[2,1-d]isothiazol mit Schmelzpunkt 51 °C. Die Ausbeute beträgt 78% der Theorie.

Beispiel 14

Analog Beispiel 13 wird Naphtho[2,1-d]isothiazol auch aus 190 Teilen 1-Chlor-2-formylnaphthalin mit 32 Teilen Schwefel hergestellt. Man erhält 125 Teile Endstoff I vom Schmelzpunkt 51 °C, was einer Ausbeute von 67,5% der Theorie entspricht.

Beispiel 15

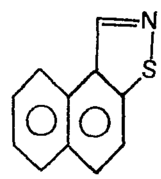

Entsprechend der Arbeitsweise von Beispiel 13 werden 245,5 Teile 2-Chlor-1-dichlormethylnaphthalin und 32 Teile Schwefel in 1500 Teilen Dimethylformamid umgesetzt. Man erhält 150 Teile Naphtho[1,2-d]isothiazol vom Schmelzpunkt 58 °C. Die Ausbeute entspricht 81% der Theorie.

Beispiel 16

In einer Rührapparatur werden 23 Teile 2,6-Dichlorbenzalchlorid und 3,2 Teile Schwefel in 300 Teilen Dimethylformamid auf 90 °C erhitzt. Während einer Stunde werden bei 90 °C 200 Teile 25-gewichtsprozentige, wässrige Ammoniaklösung langsam zugegeben. Das Gemisch wird 6 Stunden bei 90 °C weitergerührt. Die Lösung wird abgekühlt, mit 500 Teilen Wasser versetzt und dreimal mit je 100 Teilen Methylenchlorid ausgeschüttelt. Die vereinigten organischen Phasen werden getrocknet und destilliert. Bei einem Kp (2 mbar) 95 bis 100 °C werden 13 Teile 4-Chlor-1,2-benzisothiazol mit dem Schmelzpunkt 42 bis 44 °C erhalten. Die Ausbeute beträgt 80% der Theorie.

Beispiel 17

In einer Rührapparatur werden 26,2 Teile 2-Chlor-5-nitrobenzophenon und 3,2 Teile Schwefel in 160 Teilen Dimethylsulfoxid auf 70 °C erhitzt und langsam 80 Teile 25-gewichtsprozentige, wässrige Ammoniaklösung zugegeben. Das Gemisch wird 1 Stunde bei 80 °C weitergerührt, abgekühlt, mit 80 Teilen Wasser versetzt und vom ausgefallenen Feststoff abgesaugt. Man erhält 25 Teile 3-Phenyl-5-nitro-1,2-benzisothiazol mit Schmelzpunkt 135 °C. Die Ausbeute entspricht 97% der Theorie.

Beispiel 18

Analog Beispiel 17 werden 262 Teile 2-Chlor-5-nitrobenzophenon und 32 Teile Schwefel in 1600 Teilen Sulfolan umgesetzt. Nach Aufarbeitung erhält man 250 Teile 3-Phenyl-5-nitro-1,2-benzisothiazol mit Schmelzpunkt 135 °C, die einer Ausbeute von 97% der Theorie entsprechen.

Beispiel 19

Analog Beispiel 17 wird anstelle von Dimethylsulfoxid Sulfolen als Lösungsmittel verwendet. Auch hierbei werden bei einem Umsatz von 26,2 Teilen 2-Chlor-5-nitrobenzophenon 25 Teile 3-Phenyl-5-nitro-1,2-benzisothiazol erhalten, entsprechend 97% der Theorie.

Beispiel 20

Analog Beispiel 17 wird anstelle von Dimethylsulfoxid Diethylformamid als Lösungsmittel verwendet. Bei einem Umsatz von 26,2 Teilen 2-Chlor-5-nitrobenzophenon werden 25,5 Teile 3-Phenyl-5-nitro-1,2-benzisothiazol erhalten. Die Ausbeute beträgt 98% der Theorie.

Beispiel 21

Analog Beispiel 17 werden 52,5 Teile 2-Chlor-5-nitro-benzophenon und 6,4 Teile Schwefel in 400 Teilen 2,4-Dimethylsulfolan mit 200 Teilen 25-gewichtsprozentiger, wässriger Ammoniaklösung umgesetzt. Nach Aufarbeitung werden 22 Teile 3-Phenyl-5-nitro-1,2-benzisothiazol erhalten, die einer Ausbeute von 85% der Theorie entsprechen.

**Patentanspruch**

Verfahren zur Herstellung von 1,2-Benzisothiazolen der Formel

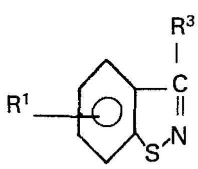

I,

worin $R^1$ Wasserstoff, einen aliphatischen, cycloaliphatischen oder aromatischen Rest, einen anellierten Phenylenrest, Halogen, eine Alkoxygruppe, Nitrogruppe oder den Rest – 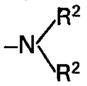 , worin die einzelnen Reste $R^2$ gleich oder verschieden sein können und jeweils Wasserstoff, einen aliphatischen, cycloaliphatischen, araliphatischen oder aromatischen Rest bedeuten, bezeichnet, und $R^3$ für Wasserstoff, einen aromatischen oder heterocyclischen Rest steht, durch Umsetzung von o-Halogenarylmethylverbindungen der Formel

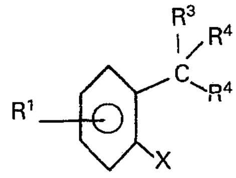

II,

in der X für Halogen steht, $R^1$ und $R^3$ die vorgenannte Bedeutung haben, die beiden Reste $R^4$ gleich oder verschieden sein können und jeweils ein Halogenatom bezeichnen oder zusammen für eine Oxogruppe stehen, mit Ammoniak und elementarem Schwefel in Gegenwart eines organischen Lösungsmittels bei erhöhter Temperatur umsetzt, dadurch gekennzeichnet, dass die Umsetzung mit 18 bis 31-gewichtsprozentigen, wässrigen Ammoniaklösungen bei Drücken von 0,8 bis 1,2 bar und Temperaturen von 65 bis 110 °C in Gegenwart von disubstituierten Sulfoxiden, Sulfonen und/oder Formamiden als Lösungsmittel durchgeführt wird.

**Revendication**

Precédé de préparation de 1,2-benzoisothiazoles de formule

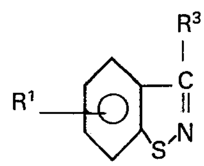

I,

dans laquelle $R^1$ représente l'hydrogène, un reste aliphatique, cycloaliphatique ou aromatique, un reste phénylène condensé, un halogène, un groupe alcoxy, nitro ou le reste 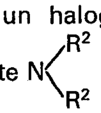 dans lequel les deux symboles $R^2$, ayant des significations identiques ou différentes, représente chacun l'hydrogène, un reste aliphatique, cycloaliphatique, araliphatique ou aromatique, et $R^3$ représente l'hydrogène, un reste aromatique ou hétérocyclique, par réaction de composés o-halogénoarylméthyliques de formule

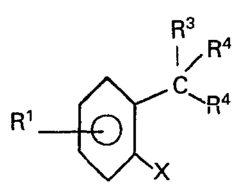

II,

dans laquelle X représente un halogène, $R^1$ et $R^3$ ont les significations indiquées ci-dessus, les deux symboles $R^4$, ayant des significations identiques ou différentes, représente chacun un atome d'halogène ou forment ensemble un groupe oxo, avec l'ammoniac et le soufre élémentaire en présence d'un solvant organique à chaud, caractérisé en ce que la réaction est effectuée avec des solutions aqueuses d'ammoniac à des concentrations de 18

à 31% en poids, sous des pressions de 0,8 à 1,2 bar et à des températures de 65 à 110 °C en présence de sulfoxydes, sulfones, et/ou formamides disubstitués qui servent de solvants.

**Claim**

A process for the preparation of a 1,2-benzisothiazole of the formula

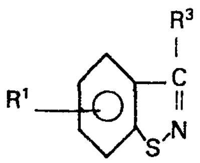

I,

where $R^1$ is hydrogen, an aliphatic, cycloaliphatic or aromatic radical, a fused phenylene, halogen, alkoxy, nitro or 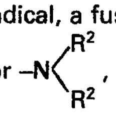, where the individual $R^2$'s may be identical or different and each denotes hydrogen, an aliphatic, cycloaliphatic, araliphatic or aromatic radical, and $R^3$ is hydrogen or an aromatic or heterocyclic radical, by reacting an o-haloarylmethyl compound of the formula

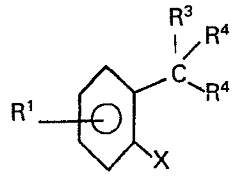

II,

where X is halogen, $R^1$ and $R^3$ have the above meanings, the two $R^4$'s may be identical or different and are each halogen or together denote an oxo group, with ammonia and elementary sulphur in the presence of an organic solvent at elevated temperature, wherein the reaction is carried out with 18 to 31% strength by weight aqueous ammonia solution at a pressure of from 0.8 to 1.2 bar and a temperature of from 65 to 110 °C in the presence of a disubstituted sulphoxide, sulphone or formamide, or a mixture thereof, as solvent.